# EUROPEAN PATENT SPECIFICATION

(11) **EP 3 139 168 B1**
(45) Date of publication and mention of the grant of the patent: **12.12.2018**
(21) Application number: 15785419.1
(22) Date of filing: 13.04.2015
(51) Int. Cl.: G01N 33/573, G01N 21/82, G01N 33/543, G01N 33/545

(54) **METHOD FOR ASSAYING CREATINE KINASE MB ISOZYME AND KIT TO BE USED THEREIN**
VERFAHREN ZUM TESTEN VON KREATINKINASE-MB-ISOZYM UND KIT ZUR VERWENDUNG DAMIT
PROCÉDÉ DE DOSAGE DE LA CRÉATINE-KINASE MB ISOENZYME ET KIT ASSOCIÉ

(30) Priority: 30.04.2014 JP 2014093351
(43) Date of publication of application: 08.03.2017
(73) Proprietor: FUJIFILM Wako Pure Chemical Corporation, Osaka-shi, Osaka 540-8605 (JP)
(72) Inventor: HANAI, Kazuma, Hyogo 661-0963 (JP); ODAGAKI, Shinichi, Hyogo 661-0963 (JP); MASUDA, Tsutomu, Hyogo 661-0963 (JP)
(74) Representative: Winter, Brandl, Fürniss, Hübner, Röss, Kaiser, Polte - Partnerschaft mbB
(86) International application number: PCT/JP2015/061350
(87) International publication number: WO 2015/166790

(56) References cited:
- WO-A1-86/00992
- WO-A1-87/03094
- DE-A1- 4 314 254
- JP-A- S62 157 571
- JP-B2- 2 774 875
- Uri Piran ET AL: "ImmunochemiluminometricAssay of Creatine Kinase MB with a MonoclonalAntibody to the MB Isoenzyme", CLIN. CHEM. CLINICAL CHEMISTRY, 1 January 1987 (1987-01-01), pages 1517-1, XP055401211, Retrieved from the Internet: URL:http://clinchem.aaccjnls.org/content/c linchem/33/9/1517.full.pdf
- U Würzburg ET AL: "Quantitative Determination of Creatine Kinase Isoenzyme Catalytic Concentrations in Serum Using Immunological Methods 1 )", J. Clin. Chem. Clin. Biochem, 1 January 1977 (1977-01-01), pages 131-137, XP055401539, Retrieved from the Internet: URL:http://citeseerx.ist.psu.edu/viewdoc/d ownload?doi=10.1.1.632.7534&rep=rep1&type= pdf
- SHIN'ICHI ODAGAKI: 'Latex Gyoshuho ni yoru Shinki na Seikagaku Jido Bunseki Sochi-yo Shiyaku 'L-type Wako CK-MB mass' ni Tsuite' JAPANESE ASSOCIATION OF MEDICAL TECHNOLOGISTS KANKOSHIN SHIBU IGAKU KENSA GAKKAI PROGRAM KOEN SHOROKUSHU vol. 50 TH, 2013, page 69, XP008185013

### SEQUENCE LISTING

<110> Wako Pure Chemical Industries, Ltd.
<120> Methd of detecting CK-MB
<130> F1984WAKOPAT
<150> JP2014-093351
   <151> 2014-04-30
<160> 2
<170> PatentIn version 3.5
<210> 1
   <211> 100
   <212> PRT
   <213> Homo sapiens
<400> 1
   Met Pro Phe Ser Asn Ser His Asn Ala Leu Lys Leu Arg Phe Pro Ala
   1               5                   10                  15
   Glu Asp Glu Phe Pro Asp Leu Ser Ala His Asn Asn His Met Ala Lys
               20                  25                  30
   Val Leu Thr Pro Glu Leu Tyr Ala Glu Leu Arg Ala Lys Ser Thr Pro
           35                  40                  45
   Ser Gly Phe Thr Leu Asp Asp Val Ile Gln Thr Gly Val Asp Asn Pro
       50                  55                  60
   Gly His Pro Tyr Ile Met Thr Val Gly Cys Val Ala Gly Asp Glu Glu
   65                  70                  75                  80
   Ser Tyr Glu Val Phe Lys Asp Leu Phe Asp Pro Ile Ile Glu Asp Arg
                   85                  90                  95
   His Gly Gly Tyr
               100
<210> 2
   <211> 381
   <212> PRT
   <213> Homo sapiens
<400> 2
   Met Pro Phe Ser Asn Ser His Asn Ala Leu Lys Leu Arg Phe Pro Ala
   1               5                   10                  15
   Glu Asp Glu Phe Pro Asp Leu Ser Ala His Asn Asn His Met Ala Lys
               20                  25                  30
   Val Leu Thr Pro Glu Leu Tyr Ala Glu Leu Arg Ala Lys Ser Thr Pro
           35                  40                  45
   Ser Gly Phe Thr Leu Asp Asp Val Ile Gln Thr Gly Val Asp Asn Pro
       50                  55                  60
   Gly His Pro Tyr Ile Met Thr Val Gly Cys Val Ala Gly Asp Glu Glu
   65                  70                  75                  80
   Ser Tyr Glu Val Phe Lys Asp Leu Phe Asp Pro Ile Ile Glu Asp Arg
                   85                  90                  95
   His Gly Gly Tyr Lys Pro Ser Asp Glu His Lys Thr Asp Leu Asn Pro
               100                 105                 110
   Asp Asn Leu Gln Gly Gly Asp Asp Leu Asp Pro Asn Tyr Val Leu Ser
           115                 120                 125
   Ser Arg Val Arg Thr Gly Arg Ser Ile Arg Gly Phe Cys Leu Pro Pro
       130                 135                 140
   His Cys Ser Arg Gly Glu Arg Arg Ala Ile Glu Lys Leu Ala Val Glu
   145                 150                 155                 160
   Ala Leu Ser Ser Leu Asp Gly Asp Leu Ala Gly Arg Tyr Tyr Ala Leu
                   165                 170                 175
   Lys Ser Met Thr Glu Ala Glu Gln Gln Gln Leu Ile Asp Asp His Phe
               180                 185                 190
   Leu Phe Asp Lys Pro Val Ser Pro Leu Leu Leu Ala Ser Gly Met Ala
           195                 200                 205
   Arg Asp Trp Pro Asp Ala Arg Gly Ile Trp His Asn Asp Asn Lys Thr
       210                 215                 220
   Phe Leu Val Trp Val Asn Glu Glu Asp His Leu Arg Val Ile Ser Met
   225                 230                 235                 240
   Gln Lys Gly Gly Asn Met Lys Glu Val Phe Thr Arg Phe Cys Thr Gly
                   245                 250                 255
   Leu Thr Gln Ile Glu Thr Leu Phe Lys Ser Lys Asp Tyr Glu Phe Met
               260                 265                 270
   Trp Asn Pro His Leu Gly Tyr Ile Leu Thr Cys Pro Ser Asn Leu Gly
                   275                 280                 285
   Thr Gly Leu Arg Ala Gly Val His Ile Lys Leu Pro Asn Leu Gly Lys
       290                 295                 300
   His Glu Lys Phe Ser Glu Val Leu Lys Arg Leu Arg Leu Gln Lys Arg
   305                 310                 315                 320
   Gly Thr Gly Gly Val Asp Thr Ala Ala Val Gly Gly Val Phe Asp Val
                   325                 330                 335
   Ser Asn Ala Asp Arg Leu Gly Phe Ser Glu Val Glu Leu Val Gln Met
               340                 345                 350
   Val Val Asp Gly Val Lys Leu Leu Ile Glu Met Glu Gln Arg Leu Glu
           355                 360                 365
   Gln Gly Gln Ala Ile Asp Asp Leu Met Pro Ala Gln Lys
       370                 375                 380

## Description

### TECHNICAL FIELD

The present invention relates to a method for measuring creatine kinase MB isozyme and a kit to be used therein.

### BACKGROUND ART

Creatine kinase (hereinafter abbreviated as CK) is an enzyme contained in skeletal muscle, cardiac muscle, smooth muscle, brain or the like, and exerts an important role in relation to energy metabolism of a cell.

CK is a dimeric protein composed of two subunits, and the subunit has two kinds: type M (muscle type, M) and type B (brain type, B). In addition, by combination of the subunits, there exists three kinds of isozymes in CK; CK-MM which is abundant in skeletal muscle, CK-MB which is abundant in cardiac muscle, and CK-BB which is abundant in brain. Among them, CK-MB has high specificity for cardiac muscle, and is released from heart muscle cell and flows out into blood, when myocardial injury occurs. Therefore, CK-MB is an important item in clinical examination as a marker of myocardial injury (myocardial infarction).

As quantitative measurement of CK-MB, an immunological inhibition method for measuring "enzyme activity", a latex turbidimetry for measuring "quantity of protein", an electrochemical luminescence immunoassay method or the like are known, and also, "an electrophoretic method" has been known as technique for analyzing isozyme.

Among these methods, the immunological inhibition method is generally used because the method can measure using a general-purpose automated analyzer. However, when CK-BB or the like is contained in a sample to be tested, an activity of the CK-BB or the like is also measured in the method, and specificity for measurement of CK-MB in the sample becomes law. Therefore, the method has a problem that correct measurement value of CK-MB cannot be obtained by the method. The electrochemical luminescence immunoassay method is a method capable of specifically measuring CK-MB without being influenced by CK-BB, however, the method has such problems that special measurement equipment is required, and reagents to be used are expensive and the like. In addition, the electrophoretic method is not utilized for the purpose of quantitative measurement of CK-MB, because of lack of rapidity, inferior quantitatively and the like.

On the other hand, the latex turbidimetry which measures a quantity of protein is a method for quantifying CK-MB as an quantity of protein by an immunological method using a monoclonal antibody specific for CK-MB. This method is capable of carrying out measurement using a general- purpose automated analyzer, and has higher precision compared with the immunological inhibition method which measures an enzyme activity, and the method has excellent specificity for CK-MB, and thus, it has been said to be a method capable of correctly monitoring quantity of released CK-MB.

Practically, however, when CK-MB value of a test sample in which CK-BB coexists is measured by the conventional latex turbidimetry, CK-BB was also measured (risk of false high value), and there has been a case where correct measurement value of CK-MB was not obtained. As a cause, it was considered that a part of the anti-CK-MB antibody to be used might have bound (cross-reacted) also to CK-BB in the sample.

Accordingly, to avoid this problem, an anti-CK-MB monoclonal antibody which does not bind to CK-MM nor CK-BB was developed, and the latex turbidimetry using this has been proposed (PATENT LITERATURE 1). This method is that a first anti-CK-MB antibody having specificity as described above and the antibody is immobilized on a latex particle, and a second anti-CK-MB antibody which binds to an epitope different from that of the first anti-CK-MB antibody immobilized on the latex particle and also has specificity described above are reacted with CK-MB in a sample to measure any increase in aggregation resulting from the antigen-antibody reaction. Practically, however, even when CK-MB was measured by this method, there was a case where false high value was obtained.

Another measurement method by the latex turbidimetry includes a method for using latex particle having immobilized a monoclonal antibody which specifically binds to CK-MB thereon, and latex particle having immobilized a monoclonal antibody which specifically binds to CK-BB thereon (PATENT LITERATURE 2). This method is the one intending to avoid an influence of CK-BB in measuring CK-MB by regulating particle size of the latex particle. Even by this method, however, it was not possible to sufficiently avoid an influence of CK-BB in measuring CK-MB.

### CITATION LIST

### Patent Literature

PATENT LITERATURE 1: JP-B-2774875
PATENT LITERATURE 2: JP-A-2013-125005

### SUMMARY OF THE INVENTION

### Technical Problem

As described above, the conventional methods for measuring CK-MB by way of measuring quantity of protein was difficult to specifically measure CK-MB with avoiding an influence of CK-BB. As the cause, it was supposed that since the specificity of the anti-CK-MB antibody might insufficient, the anti-CK-MB antibody cross-reacts not only with CK-MB but also somewhat with CK-BB (emergence of false high value).

Accordingly, the present invention has been made in view of such circumstances as described above, and an object is to provide a method for specifically measuring CK-MB with avoiding an influence of CK-BB,

### Solution to Problem

The present invention has been made for the purpose of solving the above-described problems, and comprises the following composition.
(1) A method for measuring CK-MB in a sample, in the order as claimed, comprising:
   1) a step for reacting the sample with a first antibody against CK-MB, a second antibody against CK-MB which recognizes an epitope different from that recognized by the first antibody, and an anti-CK-B antibody which recognizes a region of the 1st to 100th amino acid from N-terminus in the amino acid sequence of the subunit B of creatine kinase, wherein the first antibody and the second antibody reacts not only with CK-MB but also with a creatine kinase BB isozyme (hereinafter abbreviated as CK-BB) (step 1);
   2) a step for measuring an optical change generated by the reaction (step 2); and
   3) a step for determining a quantity of CK-MB in the sample on the basis of the result obtained in the step 2 (step 3).
(2) A method for avoiding an influence of CK-BB in measuring CK-MB with a first antibody against CK-MB and a second antibody against CK-MB which recognizes an epitope different from that recognized by the first antibody, wherein the first antibody and the second antibody reacts not only with CK-MB but also with CK-BB, comprising treating a sample with an anti-CK-B antibody which recognizes a region of the 1st to 100th amino acid from N-terminus in the amino acid sequence of the subunit B of CK.
(3) A kit for measuring CK-MB comprising a first antibody against CK-MB, a second antibody against CK-MB which recognizes an epitope different from that recognized by the first antibody, wherein the first antibody and the second antibody reacts not only with CK-MB but also with CK-BB, and an anti-CK-B antibody which recognizes a region of the 1st to 100th amino acid from N-terminus in the amino acid sequence of the subunit B of CK.

The present inventors have found that when an antibody which recognizes a specific region of subunit B of CK (anti-CK-B antibody involved in the present invention) is coexisted in the measuring system of CK-MB based on the conventional method for measuring quantity of protein, the measurement of CK-MB can be carried out with high precision and high sensitivity with avoiding an influence of CK-BB in the sample, and have thus completed the present invention.

### Advantageous Effects of invention

According to the present invention, an influence of CK-BB coexisting in the sample on measurement value (emergence of false high value) in measuring CK-MB can be avoided (suppressed); and the measurement of CK-MB can be carried out in higher precision and in higher sensitivity. In addition, according to the method of the present invention, since the quantitative measurement of CK-MB protein using a general-purpose automated analyzer, it is not necessary to use special equipment.

Furthermore, the anti-CK-B antibody involved in the present invention is the one which avoids an influence of CK-BB in measuring CK-MB. Accordingly, the present invention provides a method for avoiding an influence of CK-BB at the time of measuring CK-MB.

### BRIEF DESCRIPTION OF DRAWINGS

Fig. 1 shows an electrophoretic pattern obtained using MAb to CK-BB (produced by Meridian Life Science, Inc.) as the anti-CK-B antibody, in Example 2.
Fig. 2 shows an electrophoretic pattern obtained using CKBB Monoclonal Antibody (produced by Roche Diagnostics K.K.) as the anti-CK-B antibody, in Example 2.

### DESCRIPTION OF EMBODIMENTS

The method for measuring CK-MB of the present invention is "A method for measuring CK-MB in a sample, comprising:
(1) a step for reacting the sample with a first antibody against creatine kinase MB isozyme (hereinafter abbreviated as CK-MB), a second antibody against CK-MB which recognizes an epitope different from that recognized by the first antibody, wherein the first antibody and the second antibody reacts not only with CK-MB but also with a creatine kinase BB isozyme (hereinafter abbreviated as CK-BB), and an anti-CK-B antibody which recognizes a region of the 1st to 100th amino acid from N-terminus in the amino acid sequence of the subunit B of creatine kinase (step 1);
(2) a step for measuring an optical change tenerated by the reaction (step 2); and
(3) a step for determining a quantity of CK-MB in the sample on the basis of the result obtained in the step 2 (step 3)."

That is, in the method of the present invention, the anti-CK-B antibody of the present invention is added to a method for measuring CK-MB using two kinds of anti-CK-MB antibodies, and thereby an influence of CK-BB can be avoided, and as a result, CK-MB can be measured specifically.

As to a measurement principle that an influence of CK-BB can be avoided by the present invention, the present inventors have inferred as follows.

CK-MB is a heterodimer of a subunit M and a subunit B. CK-BB is a homodimer of the subunit B.

In the case where CK-MB and CK-BB coexist in the sample, when the sample is contacted with the anti-CK-B antibody involved in the present invention, the anti-CK-B antibody involved in the present invention binds to the subunit B of CK-MB and also with the subunit B of CK-BB in the sample. Accordingly, it is conceivable that the anti-CK-B antibody involved in the present invention, which is bound to the subunit B of CK-BB, inhibits binding of the first antibody or the second antibody with CK-BB. In consequence, it is considered that in the method for measuring CK-MB involved in the present invention, an influence of CK-BB present in the sample is avoided, and CK-MB can be measured specifically and in high precision.

The first antibody against CK-MB involved in the present invention (hereinafter, it may be abbreviated simply as "the first antibody") includes an antibody that recognizes both the subunit M and the subunit B of CK-MB.

The second antibody against CK-MB involved in the present invention (hereinafter, it may be abbreviated simply as "the second antibody") includes an antibody which recognizes both the subunit M and the subunit B of CK-MB, but recognizes an epitope different from that the first antibody recognizes.

The anti-CK-B antibody involved in the present invention includes an antibody which recognizes a region of the 1st (that is, N-terminal amino acid) to 100th amino acid from N-terminus in the amino acid sequence of the subunit B of CK. That is, only the anti-subunit B antibody, which recognizes the subunit B of CK and recognizes a specific region in the region of the 1st to 100th amino acid from N-terminus of the subunit B, can achieve an advantageous effect of the present invention.

It should be noted that, as for the anti-CK-B antibody involved in the present invention, the one which recognizes a three-dimensional structure (conformation) of a region of the 1st to 100th amino acid from N-terminus in the amino acid sequence of the subunit B of native CK is more preferable.

The amino acid sequence of the region of the 1st to 100th amino acid from N-terminus in the amino acid sequence of the subunit B of CK is specifically an amino acid sequence shown in SEQ ID NO: 1.

It should be noted that, the entire amino acid sequence of the subunit B of CK is publicly known (NCBI Reference Sequence: NP_001814.2, SEQ ID NO: 2 on the present description).

Hereinafter, the first antibody involved in the present invention, the second antibody involved in the present invention, and the anti-CK-B antibody involved in the present invention may be abbreviated collectively and simply as "the antibody involved in the present invention."

Each antibody involved in the present invention may be any one as long as it has each property as described above, and may be commercially available product or those appropriately prepared by a conventional method, and may each be a polyclonal antibody or a monoclonal antibody.

In the case where the antibody involved in the present invention is a monoclonal antibody, its origin is not especially limited, and commercially available products or the one produced by a method known per se utilizing cell fusion technique or gene recombination technique or the like [Eur. J. immunol., 6, 511 (1976)], and those having properties such as described above are all usable.

In the case where the antibody involved in the present invention is a polyclonal antibody, its origin is not especially limited, and includes, for example, the one derived from rabbit, rat, mouse, sheep, goat, horse or the like, and having properties such as described above. Commercially available products or those obtained by the method described, for example, in "T. Matsuhashi et. al., Introduction to Experimental Immunology, 2nd ed., 1981, Japan Scientific Society Press", and the like, may be used.

In addition, in the antibody involved in the present invention, the so-called antibody fragment such as Fab fragment obtained by partial digestion with papain, F(ab')₂ fragment obtained by partial digestion with pepsin, and Fab' obtained by reduction treatment of F(ab')₂ are also included.

The antibody involved in the present invention can be obtained by a method known per se, such as described above, but commercially available products may also be used.

As for the commercially available products of the first antibody and the second antibody, those which recognize different epitopes each other are selected and used from those commercially available products as the anti-CK-MB antibody, and are not especially limited.

As for the commercially available product of the anti-CK-B antibody involved in the present invention, the one which recognizes the region of the 1st to 100th amino acid from N-terminus in the amino acid sequence of the subunit B of CK may be selected and used from those commercially available as the anti-CK-B antibody or the anti-CK-BB antibody. Such commercially available product includes, for example, MAb to CK-BB which is produced by Meridian Life Science, Inc., CKBB Monoclonal Antibody which is produced by Roche Diagnostics K. K., CK-BB Monoclonal Antibody which is produced by BiosPacific, Inc., CKBB antibody which is produced by Fitzgerald I. I. and the like.

The first antibody and the second antibody may be immobilized (supported) on an insoluble carrier and used. It is convenient to use a commercially available kit containing the first antibody-immobilized latex particle and the second antibody-immobilized latex particle as constituent reagents. Such a commercially available kit includes, for example, L-type WAKO CK-MB mass (produced by Wako Pure Chemical Industries, Ltd.) or the like.

In the case where the first antibody and the second antibody are used by immobilizing on the insoluble carrier, as the insoluble carrier to be used, anyone can be used as long as it is the carrier usually used in this field, and includes as the preferable insoluble carrier the one prepared from a material such as a synthetic polymer compound such as polystyrene, polyacrylic acid, polymethacrylic acid, polyacrylamide, polyglycidyl methacrylate, polypropylene, polyvinyl chloride, polyethylene, polychlorocarbonate, a silicone resin, and silicone rubber; inorganic substances such as porous glass, frosted glass, alumina, silica gel, activated carbon, and metal oxide. In addition, these carriers can be used in various forms such as latex particle, bead, tube, disc type chip, and micro particle.

Among the above-described insoluble carriers, the latex particle is particularly preferable from the viewpoint that the chemical modification of the carrier surface according to a purpose is easy since it is artificial carrier , and non-specific reaction hardly takes place, etc. The material thereof is not especially limited as long as it is usually used in this field, and includes, for example, styrene type latex particle such as polystyrene latex particle, acrylic acid-type latex particles and the like as preferable ones. It should be noted that, among these latex particles, the polystyrene latex particle prepared by emulsion polymerization without using of an emulsifying agent is particularly preferable, because it has such property as smoothly adsorbing protein or peptide due to having strong hydrophobicity of the surface, and stably dispersing in a solution even in the absence of the emulsifying agent based on repulsion of negative charges themselves on the surface. In addition, various modified latex particle (for example, a carboxylic acid modified latex particle produced by introducing a carboxyl group to the above-described polystyrene), magnetic latex particle (a magnetic particle-encapsulated latex particle) and the like can be used as well.

Materials of the latex particle to be used for immobilizing the first antibody and the latex particle to be used for immobilizing the second antibody may be the same or each different, as long as they are those as described above.

In addition, as for the latex particle involved in the present invention, commercially available ones may be used, and those having large surface area per unit weight are preferable, because an antibody can be supported efficiently. Specifically, the average particle diameter is usually 0.05 to 2.4 µm, preferably 0.05 to 1.0 µm, and still more preferably 0.05 to 0.50 µm.

The latex particle for immobilizing the first antibody and the latex particle for immobilizing the second antibody may have the same particle size, or those having different average particle size selected from the above-described range may be used in combination.

In addition, the latex particles used for immobilizing the first antibody may be those having the same or different particle size. The latex particles used for immobilizing the second antibody may also be those having the same or different particle size.

Immobilization of the first antibody and the second antibody on the insoluble carrier may be carried out by contacting each antibody with the insoluble carrier, and an immobilization method known per se, for example, a method for immobilization by covalent anchoring, a method for immobilization by physical adsorption or the like, may be utilized.

The quantity of the first antibody to be supported on the insoluble carrier, and the quantity of the second antibody to be supported on the insoluble carrier are different depending on the carrier, and it is prepared to give usually 0.5 to 2000 mgAb, and preferably 2 to 500 mgAb, relative to 1 g of the carrier. In the case where the carrier is latex particle, it is usually 0.5 to 2000 mgAb, and preferably 2 to 500 mgAb, relative to 1 g of the latex particles.

The first antibody-immobilized insoluble carrier and the second antibody-immobilized insoluble carrier may be the one that only the first antibody is immobilized and the one that only the second antibody is immobilized. Or it may be the one that both of the first antibody and the second antibody are immobilized on the same insoluble carrier.

In the present invention, the first antibody and the second antibody may be used as the one bound to the insoluble carrier. However, the anti-CK-B antibody involved in the present invention is not used as the one bound to the insoluble carrier. For example, when the method for measuring CK-MB of the present invention is carried out using the latex particle as the insoluble carrier, a crosslinked structure will be formed among CK-MB in a sample, the first antibody immobilized on the latex and the second antibody immobilized on the latex, and thereby optical change occurs. However, although the anti-CK-B antibody involved in the present invention binds to the subunit B of CK-MB in the sample, it does not involved in formation of the crosslinked structure.

The step 1 of the method for measuring CK-MB of the present invention is a step for reacting the sample containing CK-MB with the first antibody, the second antibody, and the anti-CK-B antibody involved in the present invention.

In the step 1, it is enough for a solution containing the sample, the first antibody, the second antibody and the anti-CK-B antibody involved in the present invention is obtained eventually, however, it is preferable that the anti-CK-B antibody involved in the present invention is added before the sample is contacted and reacted with at least one of the first antibody and second antibody. In other words, it is preferable that the sample is contacted with the anti-CK-B antibody involved in the present invention in advance, and then contacted with the first antibody and the second antibody.

In the step 1, in many cases, the first antibody, the second antibody and the anti-CK-B antibody involved in the present invention are used in a solution form by making them suspended in a buffer solution or the like. The buffer solution to be used for preparing such a reagent solution is not especially limited, as long as it is the one usually used in this field, and includes the one having buffering action at a near neutral value having a pH of usually 5.0 to 10.0, and preferably 6.5 to 8.5. Specifically, it includes, for example, an HEPES buffer solution, a boric acid buffer solution, a Tris buffer solution, a phosphoric acid buffer solution, a Veronal buffer solution, a Good buffer solution or the like. In addition, buffer agent concentration of such a buffer solution is selected as appropriate from a range of usually 10 to 1000 mM, and preferably 10 to 300 mM.

In addition, in the case of using a commercially available kit such as described above containing the first antibody-immobilized latex particle or the second antibody-immobilized latex particle as a constituent reagent, a buffer solution attached to the kit may be used.

When the measurement is carried out using a first reagent containing the anti-CK-B antibody involved in the present invention and a second reagent containing the first antibody and the second antibody by a two-reagents system as will be described later, pH of the first reagent is preferably about pH 6.0 to 8.0 in consideration of stability. Taking stability into consideration, pH of the second reagent is preferably about 6.0 to 8.0 as well as the first reagent.

When a reagent solution is prepared by suspending the anti-CK-B antibody involved in the present invention in the buffer solution as described above, the concentration of the anti-CK-B antibody involved in the present invention in the reagent solution may be such level that provides an objective concentration range when the sample is mixed with the solution containing the anti-CK-B antibody involved in the present invention. For example, it may be 0.1 to 200 µg Ab/mL, preferably 0.5 to 50 µg Ab/mL, more preferably 1 to 40 µg Ab/mL, and further preferably 1 to 20 µg Ab/mL. That is, the lower limit is 0.1 µg Ab/mL, preferably 0.5 µg Ab/mL, and more preferably 1 µg Ab/mL. In addition, the upper limit thereof is 200 µg Ab/mL, preferably 50 µg Ab/mL, more preferably 40 µg Ab/mL, and further preferably 20 µg Ab/mL.

In addition, the concentration of the first antibody and/or the second antibody in the reagent solution, where the first antibody and/or the second antibody are suspended in the buffer solution as described above, is different depending on the concentration of CK-MB to be measured, and it is usually 0.4 to 4200 µg/mL, preferably 2 to 4200 µg/mL, and more preferably 2 to 420 µg/mL.

In addition, content of each insoluble carriers in the reagent solution, where the first antibody-immobilized insoluble carrier and the second antibody-immobilized insoluble carrier are suspended in the buffer solution as described above, is different depending on the carrier type to be used, and it is usually 0.001 to 10 w/v %, and preferably 0.005 to 5 w/v %. When the carrier is the latex particle, it is usually 0.1 to 10 w/v %, and preferably 0.2 to 5 w/v %.

It should be noted that, the solution containing the antibody involved in the present invention may still contain a sensitizer, a surfactant, a preservative (for example, sodium azide, salicylic acid, benzoic acid or the like), a stabilizing agent (for example, albumin, globulin, water-soluble gelatin, sugars or the like), an activator, an agent for avoiding an influence of coexisting substances, or the one used in this field which does not inhibit stability of the coexisting agent and does not inhibit an antigen-antibody reaction. In addition, the concentration ranges of these reagents and the like may also be selected appropriately from the range of the concentration and the like commonly used in the measuring method known per se.

In the step 1, when the sample is contacted/ reacted with the anti-CK-B antibody involved in the present invention, the concentration of the anti-CK-B antibody involved in the present invention in the reaction solution may be any concentration that is capable of avoiding an influence of CK-BB in measuring CK-MB by suppressing/avoiding the reaction of the anti-CK-MB antibody with CK-BB in the sample. For example, it may be 0.1 to 200 µg Ab/mL, preferably 0.5 to 50 µg Ab/mL, more preferably 1 to 40 µg Ab/mL, and further preferably 1 to 20 µg Ab/mL. That is, the lower limit is 0.1 µg Ab/mL, preferably 0.5 µg Ab/mL, and more preferably 1µg Ab/mL. In addition, the upper limit thereof is 200 µg Ab/mL, preferably 50 µg Ab/mL, more preferably 40 µg Ab/mL, and further preferably 20 µg Ab/mL.

When the sample is contacted/ reacted with the first antibody and the second antibody in the step 1, the concentration of the first antibody and the second antibody in the reaction solution at the start of the reaction may differ depending on the concentration of CK-MB in the sample, and is not especially limited, and it is usually 0.1 to 1000 µgAb /mL, preferably 0.5 to 1000 µg Ab/mL, and more preferably 0.5 to 100 µg Ab/mL.

In addition, in the case of using the latex particle as the insoluble carrier, and at the time when the sample is contacted/ reacted with the first antibody-immobilized latex and the second antibody-immobilized latex in the step 1, the concentration of the latex in the reaction solution is 0.01 to 3 w/v %, and preferably 0.01 to 1.2 w/v %.

In addition, pH at the time of the reaction of the anti-CK-B antibody involved in the present invention, the first antibody and the second antibody is not especially limited, as long as it is within a range not suppressing the antigen-antibody reaction, and includes a range of usually pH 6.0 to 10.0, and preferably 6.0 to 8.0. Temperature at the time of the reaction is also not especially limited as long as it is within a range not suppressing the antigen-antibody reaction, and includes a range of usually 10 to 50°C, and preferably 20 to 40°C. Since the reaction time thereof is different depending on the antibody involved in the present invention to be used and reaction conditions such as pH and temperature, they may be subjected to a reaction for about 1 to 60 minutes, and preferably for about 1 to 15 minutes depending on each reaction condition.

Specific methods of the step 1 include, for example, those described below.
(i) A method in which a solution containing the anti-CK-B antibody involved in the present invention, and a solution containing the first antibody and the second antibody are each prepared, and they are mixed with a sample in the order of the solution containing the anti-CK-B antibody involved in the present invention, and then the solution containing the first antibody and the second antibody (two-reagents system);
(ii) A method in which a solution containing the anti-CK-B antibody involved in the present invention, a solution containing the first antibody, and a solution containing the second antibody are each prepared, and after a sample is mixed with the solution containing the anti-CK-B antibody involved in the present invention, the solution containing the first antibody, and the solution containing the second antibody are mixed thereto in order (the order in which the solution containing first antibody and the solution containing second antibody are added is not predetermined, and may be added in either order.) ; and
(iii) A method in which a solution containing the first antibody, the second antibody, and anti-CK-B antibody involved in the present invention is prepared, and these components are reacted at a time by mixing the solution with the sample.

Among those methods described above, the method (i), that is, a method of reacting sample containing CK-MB with the anti-CK-B antibody involved in the present invention, and then reacting with the first antibody and the second antibody is preferable, in consideration of the case where the measurement is carried out using an automated analyzer, and working efficiency of the measurement, etc.

It should be noted that, a pretreatment solution for a sample such as an avoidance agent of an influence of coexisting materials in the sample may be brought into contact with the sample prior to the sample being brought into contact with the antibody involved in the present invention.

In addition, in any one of the reagents constituting a commercially available kit for measuring CK-MB using the anti-CK-MB antibody which is usually used in the field of Clinical Examination, the anti-CK-B antibody involved in the present invention may be present and used, so as to attain any of the above-described state.

The step 2 of the method for measuring CK-MB of the present invention is a step for measuring an optical change generated by the reaction of the step 1.

In this step 2, measurement of optical change generated by the reaction may be carried out according to the measurement method using the anti-CK-MB antibody known per se.

The optical change includes absorbance change, turbidity change, scattered light intensity change or the like.

Measurement of optical change means to measure optical change resulting from immune aggregation, and includes specifically, an immune agglutination method such as an immunoturbidimetry, and an immunological nephelometry. These measurement methods may be carried out in accordance with a method known per se. For example, in the case of using the immunoturbidimetry, it may be carried out in accordance with the method described in "Outline of Clinical Examination Methods", 30th ed., Kanehara & Co., Ltd., pp. 853-854" or the like; and in the case of using the immunological nephelometry, it may be carried out in accordance with the method described in "Outline of Clinical Examination Methods, 30th ed., Kanehara & Co., Ltd., pp. 851-853" or the like.

Among them, measurement by the immunoturbidimetry is preferable, and particularly the latex turbidimetry using the latex particle is preferable in the viewpoint that it has high measurement sensitivity; it can be measured using a general-purpose automated analyzer; it is convenient; and it is capable of further enjoying advantageous effects of the present invention.

In the case where the measurement method of the present invention is carried out according to the latex turbidimetry, it may be carried out in accordance with the measurement conditions (for example, measurement wavelength and the like) and the measurement operation of the method for measuring CK-MB by the latex turbidimetry known per se, except for bringing the anti-CK-B antibody involved in the present invention is contacted and mixed with a sample.

In the case where the measurement of CK-MB is carried out by the latex turbidimetry, wavelength for absorbance measurement is usually 340 to 880 nm, and preferably 540 to 800 nm. In the case where measurement is carried out by two wavelengths, it may be measured with a main wavelength at around 540 nm, and a sub wavelength at around 800 nm.

Optical change involved in the present invention may be determined, for example, as follows:
(1) After starting the reaction of the first antibody-immobilized insoluble carrier and the second antibody-immobilized insoluble carrier with CK-MB, optical measurement of the reaction solution is carried out twice at appropriate interval, and the difference of the two measurement values is defined as optical change (end point method).
(2) Optical change rate (particularly, the maximum change rate thereof) of the reaction solution after starting the reaction of the first antibody-immobilized insoluble carrier and the second antibody-immobilized insoluble carrier with CK-MB is defined as absorbance change (rate method).

Measurement of absorbance change, turbidity change, or scattered light intensity change may be carried out manually, as matter of course. Additionally, since the method of the present invention is applicable to a measurement system using an automated analyzer, the measurement may be carried out using a biochemical equipment such as an automated analyzer, and a spectrophotometer, and a dedicated nephelometric machine such as a laser nephelometer. Detailed operation may be carried out in accordance with each device manual.

Combinations of reagents in the case of carrying out the measurement manually or by using an automated analyzer are not especially limited, and may be determined appropriately in accordance with environment of the automated analyzer to be applied, other factors and the like.

In the case where the step 2 of the method for measuring CK-MB involved in the present invention is carried out using, for example, a commercially available kit utilizing a method for measuring CK-MB by the latex turbidimetry and a general-purpose automated analyzer, the anti-CK-B antibody involved in the present invention may be contained in any one of the constituent reagents of the kit. In addition, common measurement of CK-MB using the automated analyzer may be carried out according to the instruction manual attached to the kit, except for the anti-CK-B antibody involved in the present invention is contacted or reacted with the sample simultaneously or before contacted with other antibodies.

The step 3 of the method for measuring CK-MB of the present invention is "a step for determining a quantity of CK-MB on the basis of the result obtained in the step 2."

For example, the quantity of CK-MB in the sample may be calculated by applying the quantity of optical change measured in the step 2 to a calibration curve showing the relationship between CK-MB concentration and the quantity of optical change which was prepared, for example, by carrying out in the same measurement method using standard solutions of known concentrations of CK-MB as samples in advance.

As an example of the method for measuring CK-MB of the present invention (step 1 to step 3), the case where the above-described method (i) (a method in which a solution containing the anti-CK-B antibody involved in the present invention and a solution containing the first antibody and the second antibody are each prepared, and then these solutions are mixed with a sample in the order of the solution containing the anti-CK-B antibody involved in the present invention, and then the solution containing the first antibody and the second antibody), with carrying out the measurement by immunoturbidimetry using the latex particle will be specifically explained as follows.

First, for example, a sample to be measured for CK-MB such as blood, serum, and plasma is contacted and mixed with the first reagent containing 0.1 to 200 µg Ab/mL of the anti-CK-B antibody involved in the present invention, and reacted usually at 10 to 50°C, preferably at 20 to 40°C, for usually 1 to 60 minutes, preferably for 1 to 15 minutes, and more preferably for about 5 minutes. Then, the reaction solution is mixed with the second reagent containing the first antibody immobilized on the latex particle and the second antibody immobilized on the latex particle, and reacted at usually 10 to 50°C, preferably at 20 to 40°C, for usually 1 to 60 minutes, preferably for 1 to 15 minutes, and more preferably for about 5 minutes. The resulting absorbance change of the reaction solution generated by the antigen-antibody reaction is measured, for example, at a measurement wavelength of 340 to 880 nm, and preferably 540 to 800 nm. Separately, measurement is carried out in a same manner by using, for example, CK-MB standard products of known concentrations as samples to prepare a calibration curve showing relationship between CK-MB concentration and absorbance, in advance. Then, the concentration of CK-MB is determined by applying the measured values obtained using the sample to be measured for CK-MB to the calibration curve to thereby quantify CK-MB in the sample.

A method for avoiding an influence of CK-BB in measuring CK-MB of the present invention includes a method for treating the sample by adding the anti-CK-B antibody involved in the present invention to the sample containing CK-MB. Specific examples of the reagents and the treatment method used in that occasion include those above-described in the method for measuring CK-MB in the sample.

The kit for measuring CK-MB of the present invention may be any one comprising the reagents containing the first antibody, the second antibody and the anti-CK-B antibody involved in the present invention, as constituent reagents. A preferred embodiment, a specific example and the concentration or the like of each constituent is as described in the above-described explanation regarding the method for measuring CK-MB of the present invention.

In addition, the reagents containing the first antibody, the second antibody, or the anti-CK-B antibody involved in the present invention may be those in a solution state such as a suspended solution of the antibody involved in the present invention, the first antibody-immobilized insoluble carrier, the second antibody-immobilized insoluble carrier, the anti-CK-B antibody involved in the present invention in an appropriate buffer solution, or it may be frozen products, or freeze-dried products thereof. Specific examples of the buffering agents and the like to be used for this purpose, pH and the concentration thereof are as described above.

In addition, the reagents contained in these kits may add the reagents usually used in this field, for example, a buffer agent, a sensitizer, a surfactant, a preservative (for example, sodium azide, salicylic acid, benzoic acid or the like), a stabilizing agent (for example, albumin, globulin, water-soluble gelatin, sugars or the like), an activator, an agent for avoiding an influence of coexisting substances, or the one used in this field, as long as the reagents does not interfere stability of the coexisting agent, and does not inhibit an antigen-antibody reaction. In addition, the concentration ranges of these reagents and the like may be appropriately selected from the concentration ranges and the like usually used in the measurement method known per se.

In addition, in the case where the kit is composed of a plurality of reagent solutions, the reagents necessary to measure the objective components are contained in each of the reagent solutions. These reagents may be contained by being appropriately separated in any one of the reagent solutions, so that the reaction for measuring objective components is started at the time of mixing each of the reagent solutions. The concentration of the reagents which constitute these reagent solutions may be selected appropriately from a range commonly used in this field.

Further, a standard product of CK-MB for preparing a calibration curve to be used for measuring CK-MB may be combined in the kit. As the standard, a commercially available standard product, or the one produced according to a publicly known method may also be used.

Furthermore, in the kit of the present invention, a manual etc. for use in the method for measuring CK-MB may be included. The "manual" means the instruction manual, the package insert, or brochure (leaflet), etc. of the kit, in which features, principles, operating procedures, and determination procedures, etc. of the method is described substantially in sentence or by figures and tables.

Specific embodiments of the kit of the present invention include, for example, the following constitution:
(1) the one which is constituted of the first reagent containing the anti-CK-B antibody involved in the present invention, and a second reagent containing the first antibody and the second antibody,
(2) the one which is constituted the first reagent containing the anti-CK-B antibody involved in the present invention, the second reagent containing the first antibody, and a third reagent containing the second antibody, or
(3) the one which is constituted a reagent containing the first antibody, the second antibody and the anti-CK-B antibody involved in the present invention.

The sample involved in the present invention may be any one containing CK-MB, and includes specifically body fluid, for example, blood, plasma, serum, synovial fluid, pleural fluid, lymph fluid, spinal fluid or the like, and urine, stool, saliva or the like, and among them, serum, plasma or the like is included as a preferable one.

Hereinafter, the present invention will be explained more specifically in reference to Examples, but the present invention should not be limited in any way by these Examples.

### Examples

### Example 1

Measurement was carried out using L-type Wako CK-MB mass (produced by Wako Pure Chemical Industries, Ltd.) which is a commercially available kit for measuring CK-MB, and according to the description in an attached document of the kit, as follows.

The above-described kit is the one to be used in the method for measuring CK-MB by the latex turbidimetry using the two types of latex particles, where two types of anti-CK-MB antibodies having different antigenic determinant was immobilized on each latex particle.

### (1) Preparation of CK-BB Sample

Human brain-derived purified CK-BB (Meridian Life Science, Inc.) was added to human serum so as to attain 400 ng/mL or 2000 ng/mL, and used as the CK-BB samples.

### (2) Preparation of First Reagent

As an anti-CK-B antibody, MAb to CK-BB (Meridian Life Science, made Inc.) or CKBB Monoclonal Antibody (produced by Roche Diagnostics Co., Ltd.), which was commercially available mouse anti-CK-BB monoclonal antibody, was added to buffer solution attached to the kit to prepare antibody-containing buffer solutions (the first reagents) each having the concentration of 0, 1.25, 2.5, 5, 10, 20, 40, 80, and 160 µg/mL.

It should be noted that, when the CK-BB sample and the first reagent are mixed, each of the concentration of the anti-CK-B antibody in the each reaction solutions are 0, 1.2, 2.3, 4.9, 9.4, 18.8, 37.5, 75, 150 µg/mL, respectively.

### (3) Second Reagent

The latex reagent solution attached to the kit was used as the second reagent. The latex reagent solution contains two types of latex particles, where two types of anti-CK-MB antibodies having different recognition epitope was immobilized on each latex particle.

### (4) Measurement of CK-MB (2-point end method)

Using Hitachi autoanalyzer 7170 (manufactured by the Hitachi High-Technologies Corp.), the following measurement was carried out.

That is, 150 µL of the first reagent prepared in the above-described (2) was mixed with 10 µL of a CK-BB sample prepared in the above-described (1), and after incubation at 37°C for 5 minutes, 50 µL of the second reagent was added, and incubated for further 5 minutes. Change of absorbance at 660 nm from 30 seconds to 5 minutes after adding the second reagent was measured.

As a control, CK-MB in the same sample was measured using the same reagent and the same measuring instrument by the same method, except for using a buffer solution not containing the anti-CK-B antibody as the first reagent.

### (5) Results

The results obtained in the case of using the first reagent containing MAb to CK-BB as the anti-CK-B antibody are shown in Table 1.

Table 1 shows measurement value obtained by carrying out the measurement of CK-MB using CK-BB sample containing 400 ng/mL or 2000 ng/mL of CK-BB (CK-MB measurement value), and the ratio of CK-MB measurement value obtained by carrying out the measurement of CK-MB using the CK-BB sample to the concentration of CK-BB in the CK-BB sample used for the measurement (CK-BB crossing rate).

**Table 1**

| Concentration of anti-CK-B antibody in the first reagemt (µg/ml) | 0 | 1.25 | 2.5 | 5 | 10 | 20 | 40 | 80 | 160 |
|---|---|---|---|---|---|---|---|---|---|
| **CK-MB measurement value (ng/mL)** | | | | | | | | | |
| 400ng/mL (CK-BB) | 18.6 | 9.7 | 5.2 | 3.2 | 2.4 | 2.2 | 1.9 | 2.1 | 1.9 |
| 2000ng/mL (CK-BB) | 105.2 | 62.3 | 32.1 | 15.4 | 10.3 | 5.4 | 3.2 | 2.2 | 1.7 |

| **CK-BB crossing rate** | | | | | | | | | |
|---|---|---|---|---|---|---|---|---|---|
| 400ng/mL (CK-BB) | 4.6% | 2.4% | 1.3% | 0.8% | 0.6% | 0.5% | 0.5% | 0.5% | 0.5% |
| 2000ng/mL (CK-BB) | 5.3% | 3.1% | 1.6% | 0.8% | 0.5% | 0.3% | 0.2% | 0.1% | 0.1% |

In the present Example, CK-MB value in a sample not containing CK-MB was measured. However, according to Table 1, for example, an measurement value of 18.6 ng/mL was obtained when the measurement of CK-MB was carried out using the sample containing 400 ng/mL of CK-BB and the first reagent not containing the anti-CK-B antibody (MAb to CK-BB) (the case where the addition concentration of the anti-CK-B antibody is 0 µg/mL). Since this sample contains 400 ng/mL of CK-BB, it turns out that CK-BB has also been measured at a crossing rate of 18.6 (ng/mL) / 400 (ng/mL) × 100 ≈ about 4.6%, in this measurement (false high value). Similarly, when the measurement of CK-MB was carried out using the sample containing 2000 ng/mL of CK-BB, and using the first reagent not containing anti-CK-B antibody (MAb to CK-BB), the crossing rate was 5.3%.

In contrast, when the measurement of CK-MB was carried out using the first reagent containing anti-CK-B antibody (MAb to CK-BB), the crossing rate of CK-BB was significantly reduced. For example, when the measurement of CK-MB was carried out using the first reagent containing 5 µg/mL of the anti-CK-B antibody (MAb to CK-BB), CK-BB crossing rate was 0.8% in both case of using the sample containing 400 ng/mL thereof, or the sample containing 2000 ng/mL thereof. That is, when the measurement of CK-MB was carried out by using the first reagent containing the anti-CK-B antibody (MAb to CK-BB), an influence of CK-BB on CK-MB measurement could be significantly suppressed, as compared with the case of carrying out the measurement of CK-MB using the first reagent not containing the anti-CK-B antibody (MAb to CK-BB).

Similarly, the results in the case of using the first reagent containing the CKBB Monoclonal Antibody as the anti-CK-B antibody are shown in Table 2.

**Table 2**

| Concentration of anti-CK-B antibody in the first reagemt (µg/mL) | 0 | 1.25 | 2.5 | 5 | 10 | 20 | 40 | 80 | 160 |
|---|---|---|---|---|---|---|---|---|---|
| **CK-MB measurement value (ng/mL)** | | | | | | | | | |
| 400ng/mL (CK-BB) | 18.6 | 6.5 | 3.8 | 1.9 | 2.0 | 2.2 | 1.9 | 2.1 | 2.0 |
| 2000ng/mL (CK-BB) | 105.2 | 51.5 | 20.8 | 8.5 | 6.5 | 4.2 | 3.0 | 1.5 | 1.4 |

| **CK-BB crossing rate** | | | | | | | | | |
|---|---|---|---|---|---|---|---|---|---|
| 400ng/mL (CK-BB) | 4.6% | 1.6% | 1.0% | 0.5% | 0.5% | 0.6% | 0.5% | 0.5% | 0.5% |
| 2000ng/mL (CK-BB) | 5.3% | 2.6% | 1.0% | 0.4% | 0.3% | 0.2% | 0.2% | 0.1% | 0.1% |

As is clear from the results of Table 2, in the case where the measurement of CK-MB was carried out using the first reagent containing anti-CK-B antibody (CKBB Monoclonal Antibody) also, an influence of CK-BB could be suppressed significantly, as compared with the case of carrying out the measurement of CK-MB by using the first reagent not containing the anti-CK-B antibody (CKBB Monoclonal Antibody).

As the results, it has been understood that an influence of CK-BB contained in a sample is avoided by adding the anti-CK-B antibody (CKBB Monoclonal Antibody) to the conventional CK-MB measurement system by latex turbidimetry, and the measurement with high specificity for CK-MB can be carried out.

In addition, although data were not shown, a measurement of CK-MB was carried out by the similar method as described above, except for using the first reagent containing as an anti-CK-B antibody the CK-BB Monoclonal Antibody produced by BiosPacific, Inc. or the CKBB antibody produced by Fitzgerald I. I. As a result, an influence of CK-BB could be avoided significantly, as compared with the case of carrying out a measurement of CK-MB by using the first reagent not containing these antibodies. Accordingly, it has been understood that an influence of CK-BB contained in a sample is avoided, and the measurement with high specificity for CK-MB can be carried out by adding such a CK-B antibody (CK-BB Monoclonal Antibody produced by BiosPacific, Inc. or CKBB antibody produced by Fitzgerald 1. 1.) to the conventional CK-MB measurement system by latex turbidimetry,

### Example 2

Since it was confirmed from the results of Example 1 that the use of the anti-CK-B antibody (anti-CK-B antibody involved in the present invention) used in Example 1 could avoid the influence of CK-BB during measuring CK-MB by latex turbidimetry, an epitope for these antibodies was confirmed by the following method.

### (1) Western Blotting

2 µL of 0.1 mg/mL human brain-derived purified CK-BB (Meridian Life Science, Inc.) was applied to SuperSep™ (electrophoresis gel, produced by Wako Pure Chemical Industries, Ltd.; 5-20% gradient gel) to carry out SDS-PAGE electrophoresis (at a constant current of 25 mA). Then, fraction after the electrophoresis was transferred to PVDF membrane by a semi-dry blotting method.

Block Ace (produced by DS Pharma Biomedical Co., Ltd.) was dissolved in PBS-T (Phosphate Buffered Saline with Tween™ 20, pH 7.4, produced by Wako Pure Chemical Industries, Ltd.), so as to attain the concentration of 4 w/v % to prepare a blocking solution. The above-described PVDF membrane after transfer was immersed in this blocking solution to carry out blocking treatment at room temperature for 1 hour. Then, the PVDF membrane was cut by each fraction lanes, and each of them was immersed in the new blocking solution.

Then, as the anti-CK-B antibody, 4 types of commercially available rabbit anti-CK-BB polyclonal antibodies (all produced from Abcam plc) each includingan antibody against (recognizes) an antigen that is 200aa-300aa (a moiety of the 200th to 300th amino acid from N-terminus in the entire amino acid sequence of the subunit B of CK (SEQ ID NO: 1)), an antibody against an antigen that is 350aa-C-terminus (a moiety of the 350th to C-terminus amino acid from N-terminus), an antibody against an antigen that is 165aa-357aa (a moiety of the 165th to 357th amino acid from N-terminus), and an antibody against an antigen that is 1aa-100aa (a moiety of the 1st to 100th amino acid from N-terminus), were each added to the each blocking solution where the PVDF membrane was immersed, and treated at room temperature for 2 hours.

After that, the same anti-CK-B antibody as used in Example 1 (Mab to CK-BB or CK-BB Monoclonal Antibody, that is the anti-CK-B antibody involved in the present invention), was further added to the above-described blocking solution where the membrane was immersed, and immersed the membrane at 4°C overnight. After the blocking solution was replaced with sufficient amount of PBS-T, and the membrane was immersed in the PBS-T for 10 minutes, and then the membrane was washed 3 times by the method for replacing with a fresh PBS-T. The membrane was immersed in the fresh blocking solution, and then Polyclonal Rabbit Anti-Mouse Immunoglobulins/HRP (DAKO A/S) was added to the blocking solution and immersed at room temperature for 1 hour. Next, the membrane was washed with sufficient amount of PBS-T, and light was emitted using an ECL™ Prime Western Blotting Detection Reagent (chemiluminescent substrate for peroxidase, produced by GE Healthcare), and detected it using a LAS4000 (manufactured by FUJIFILM Corporation) for an exposure period of 10 seconds.

### (2) Results

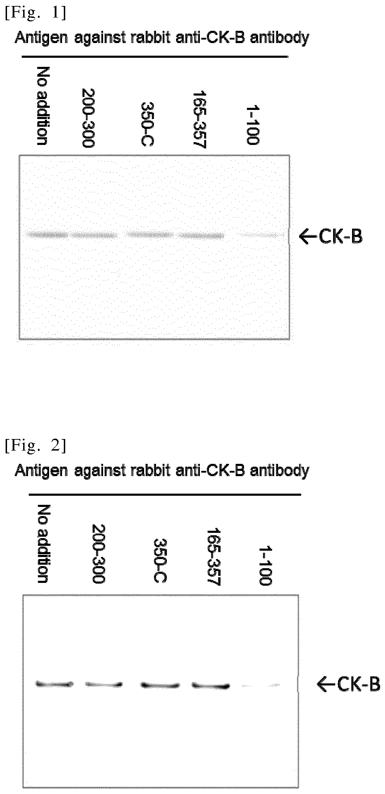

The results obtained by using MAb to CK-BB as the anti-CK-B antibody involved in the present invention are shown in Fig. 1, and the results obtained by using the CK-BB Monoclonal Antibody as the anti-CK-B antibody involved in the present invention are shown in Fig. 2, respectively.

In Fig. 1 and Fig. 2, the position of an electrophoretic fraction of CK-BB is indicated by an arrow mark.

In addition, "no addition" in Fig. 1 and Fig. 2 shows the case where "pretreatment of an electrophoretic fraction using a rabbit anti-CK-B antibody was not carried out."

Further, for example, "200-300" shows the result of "the case where, after the electrophoretic fraction was reacted with the rabbit anti-CK-B antibody against an antigen that is a moiety of the 200th to 300th amino acid from N-terminus of subunit B of CK in advance, it was further reacted with the anti-CK-B antibody involved in the present invention [MAb to CK-BB (Fig. 1) or CKBB Monoclonal Antibody (Fig. 2)]."

"350-C" shows the result of "the case where, after the electrophoretic fraction was reacted with the rabbit anti-CK-B antibody against an antigen that is a moiety of the 350th to C-terminus amino acid from N-terminus of subunit B of CK in advance, it was further reacted with the anti-CK-B antibody involved in the present invention [MAb to CK-BB (Fig. 1) or CKBB Monoclonal Antibody (Fig. 2)]."

"165-357" shows the result of "the case where, after the electrophoretic fraction was reacted with the rabbit anti-CK-B antibody against an antigen that is a moiety of the 165th to 357th amino acid from N-terminus of subunit B of CK in advance, it was further reacted with the anti-CK-B antibody involved in the present invention [MAb to CK-BB (Fig. 1) or CKBB Monoclonal Antibody (Fig. 2)]."

"1-100" shows the result of "the case where, after the electrophoretic fraction was reacted with the rabbit anti-CK-B antibody against an antigen that is a moiety of the 1st to 100th amino acid from N-terminus of subunit B of CK in advance, it was further reacted with the anti-CK-B antibody involved in the present invention [MAb to CK-BB (Fig. 1) or CKBB Monoclonal Antibody (Fig. 2)]."

As is clear from Fig. 1 and Fig. 2, in the case of "1-100", that is, only "in the case where, after the electrophoretic fraction was reacted with the rabbit anti-CK-B antibody against an antigen that is a moiety of the 1st to 100th amino acid from N-terminus of subunit B of CK in advance, it was further reacted with the anti-CK-B antibody involved in the present invention [MAb to CK-BB (Fig. 1) or CKBB Monoclonal Antibody (Fig. 2)]", the degree of staining of the fraction was significantly reduced.

This shows that the rabbit anti-CK-B antibody used (the rabbit anti-CK-B antibody against an antigen that is a moiety of the 1st to 100th amino acid from N-terminus of subunit B of CK) and the anti-CK-B antibody involved in the present invention [MAb to CK-BB and CKBB Monoclonal Antibody] have competed for the antigen (a moiety of the 1st to 100th amino acid from N-terminus of the subunit B of CK).

From the results described above, it has been clarified that the anti-CK-B antibody involved in the present invention (MAb to CK-BB and CKBB Monoclonal Antibody) is an antibody which recognizes the region of the 1st to 100th amino acid of the subunit B of CK.

### Comparative Example 1

Whether the antibodies which recognize the region other than the "region of the 1st to 100th amino acid from N-terminus" of the subunit B of CK can avoid an influence of CK-BB in the measurement of CK-MB or not was investigated according to the following method.

### (1) Preparation of CK-BB Sample

The same CK-BB sample as in Example 1 was used.

### (2) Preparation of First Reagent

By adding commercially available anti-CK-B antibodies shown below which recognize a different region each other to a buffer solution attached to the kit (L-type Wako CK-MB mass), the antibody-containing buffer solutions (the first reagents) in the concentration of 5 µg/mL were each prepared. The "antigen" against each antibody is shown on the basis of the description regarding an antigen region in the attached document of each antibody.

Antibody a: an anti-CK-B antibody which recognizes the amino acid sequence of the 200th to 300th amino acid from N-terminus of the subunit B of CK (rabbit anti-CK-BB polyclonal antibody, produced by Abcam plc).

Antibody b: an anti-CK-B antibody which recognizes the amino acid sequence of the 350th to C-terminal amino acid from N-terminus of the subunit B of CK (rabbit anti-CK-BB polyclonal antibody, produced by Abcam plc).

Antibody c: an anti-CK-B antibody which recognizes the amino acid sequence of the 165th to 357th amino acid from N-terminus of the subunit B of CK (rabbit anti-CK-BB polyclonal antibody, produced by Abcam plc).

Antibody d: MAb to CK-BB (produced by Meridian Life Science, Inc., the anti-CK-B antibody involved in the present invention used in Example 1).

### (3) Second Reagent

The same latex reagent solution attached to the kit as used in Example 1 was used as the second reagent.

### (4) Measurement of CK-MB (2-point end method)

Using the same equipment as used in Example 1, and in the same method, measurement of CK-MB was carried out.

### (5) Results

The results are shown in Table 3.

Table 3 shows measurement value obtained by carrying out the measurement of CK-MB using CK-BB sample containing 400 ng/mL or 2000 ng/mL of CK-BB (CK-MB measurement value), and the ratio of CK-MB measurement value obtained by carrying out measurement of CK-MB using the CK-BB sample to the concentration of CK-BB in the CK-BB sample used for the measurement (CK-BB crossing rate).

**Table 3**

| Anti-CK-B antibody | No addition | a | b | c | d |
|---|---|---|---|---|---|
| **CK-MB measurement value (ng/mL)** | | | | | |
| 400ng/mL (CK-BB) | 18.6 | 18.9 | 18.5 | 16.1 | 3.2 |
| 2000ng/mL (CK-BB) | 105.2 | 106.2 | 107.1 | 97.5 | 15.4 |

| **CK-BB crossing rate** | | | | | |
|---|---|---|---|---|---|
| 400ng/mL (CK-BB) | 4.6% | 4.7% | 4.6% | 4.0% | 0.8% |
| 2000ng/mL (CK-BB) | 5.3% | 5.3% | 5.4% | 4.9% | 0.8% |

As is clear from Table 3, the case where the measurement of CK-MB was carried out using the first reagent containing the antibodies a to c, there was little change in crossing rate with CK-BB, as compared with the case where the measurement of CK-MB was carried out using the first reagent not containing the antibodies a to c. Only the case where the measurement of CK-MB was carried out using the first reagent containing the antibody d (anti-CK-B antibody involved in the present invention), the crossing rate with CK-BB was significantly reduced.

As the results, it has been understood that the antibodies (antibodies a to c) which recognize the region other than the region of the 1st to 100th amino acid from N-terminus of the subunit B of CK are unable to suppress an influence of CK-BB on the measurement system of CK-MB.

### Example 3

Using L-type Wako CK-MB mass (produced by Wako Pure Chemical Industries, Ltd.) which is the same commercially available kit for measuring CK-MB as used in Example 1, and according to the method described in the attached document of the kit, the following measurement was carried out.

### (1) Measurement of CK-MB according to the Method of the Present Invention 1) Measurement Sample

Human serums where the presence of CK-BB has been confirmed were selected from the serums purchased from VERITAS Corp., NITTOBO Medical Co., Ltd., or International BioScience, Inc., and used them as the measurement sample.

### 2) Preparation of First Reagent

The anti-CK-B antibody involved in the present invention (Mab to CK-BB or CK-BB Monoclonal Antibody) was added to a buffer solution attached to the kit, and the antibody-containing buffer solution (the first reagent) having the concentration of 5 µg/mL was prepared.

It should be noted that, when the measurement sample and the first reagent were mixed, the concentration of the anti-CK-B antibody involved in the present invention in the reaction solution is 4.7 µg/mL.

### 3) Second Reagent

The same latex reagent solution attached to the kit as used in Example 1 was used as the second reagent.

### 4) Measurement of CK-MB (2-point end method)

Using a LABOSPECT008 Automatic Analyzer (manufactured by Hitachi High-Technologies Corp.), measurement was carried out. To a 7 µL of CK-MB-containing sample of the above-described 1), 100 µL of the first reagent prepared in the above-described 2) was mixed, and after incubation at 37°C for 5 minutes, 33 µL of the second reagent was added and incubated for further 5 minutes. Change of absorbance at 660 nm from 30 seconds to 5 minutes after the addition of the latex reagent solution was measured.

As a control, CK-MB in the same sample was measured using the same reagent and the same measurement instrument by the same method, except for using a buffer solution not containing the anti-CK-B antibody involved in the present invention.

### (2) Measurement of CK-MB by the Conventional Method (electrochemical luminescence immunoassay method)

Using ECLusys reagent CK-MBII (produced by Roche Diagnostics Co., Ltd.) which is a kit for measuring CK-MB by the electrochemical luminescence immunoassay method, and using Cobas 8000 (produced by Roche Diagnostics Co., Ltd.), CK-MB in the same sample was measured.

### (3) Results

The results are shown in Table 4 below.

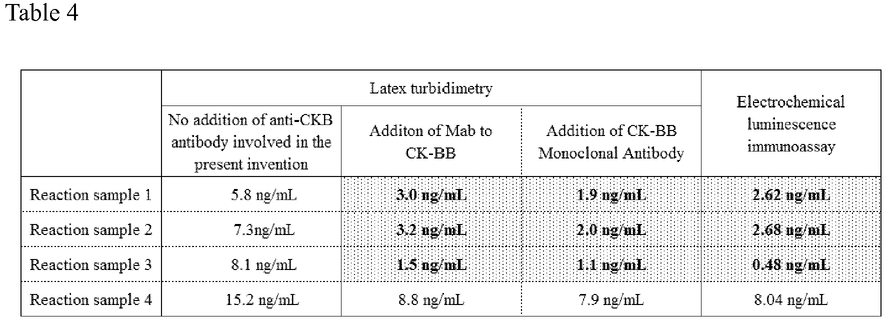

In Table 4, in the case where a CK-MB value equal to or less than the reference standard value of CK-MB protein amount "5.0 ng/mL (quoted from Outline of Clinical Examination Methods, 33rd edition)" was obtained, those were shown shaded.

As is clear from Table 4, measurement values obtained by the method of the present invention ("Addition of Mab to CK-BB" or "Addition of CKBB Monoclonal Antibody") are completely consistent in cut-off judgement with measurement value obtained by the conventional electrochemical luminescence immunoassay method, and it has been understood that reliability of measurement value obtained by the method of the present invention is higher, as compared with measurement values obtained by the conventional latex turbidimetry ("No addition of the anti-CK-B antibody").

### INDUSTRIAL APPLICABILITY

The method for measuring CK-MB of the present invention is capable of measuring CK-MB specifically by avoiding its influence, even when CK-BB coexists in the sample,. In addition, the method for measuring CK-MB of the present invention is useful in that it can be used for measuring CK-MB, in the field of clinical examination, because it is applicable to a general-purpose automated analyzer.

## Claims

1. A method for measuring creatine kinase MB isozyme (hereinafter abbreviated as CK-MB) in a sample, comprising:
(1) a step for reacting the sample with an anti-CK-B antibody which recognizes a region of the 1st to 100th amino acid from N-terminus in the amino acid sequence of an subunit B of creatine kinase, and then reacting with a first antibody against CK-MB and a second antibody against CK-MB which recognizes an epitope different from that recognized by the first antibody against CK-MB, wherein the first antibody and the second antibody reacts not only with CK-MB but also with a creatine kinase BB isozyme (hereinafter abbreviated as CK-BB) (step 1);
(2) a step for measuring an optical change generated by the reaction (step 2); and
(3) a step for determining a quantity of CK-MB in the sample on the basis of the result obtained in the step 2 (step 3) .

2. The method according to claim 1, wherein the first antibody and the second antibody recognize both a subunit M and the subunit B of CK-MB.

3. The method according to claim 1, wherein the region to be recognized by the anti-CK-B antibody is the one having an amino acid sequence shown in SEQ ID NO: 1.

4. The method according to claim 1, wherein the optical change is absorbance change, turbidity change, or scattered light intensity change.

5. The method according to claim 1, wherein the first antibody and/or the second antibody are immobilized on a insoluble carrier.

6. The method according to claim 5, wherein the insoluble carrier is latex particle.

7. A method for avoiding a influence of CK-BB in measuring CK-MB with a first antibody against CK-MB and a second antibody against CK-MB which recognizes an epitope different from that recognized by the first antibody,
wherein the first antibody and the second antibody reacts not only with CK-MB but also with CK-BB, comprising treating a sample with an anti-CK-B antibody which recognizes a region of the 1st to 100th amino acid from N-terminus in the amino acid sequence of the subunit B of creatine kinase.

8. The method according to claim 7, wherein the CK-MB measurement is carried out using a first antibody against CK-MB and a second antibody against CK-MB which recognizes an epitope different from that recognized by the first antibody, and the measurement is influenced by CK-BB.

9. A kit for measuring CK-MB comprising a first antibody against CK-MB, a second antibody against CK-MB which recognizes an epitope different from that recognized by the first antibody, wherein the first antibody and the second antibody reacts not only with CK-MB but also with CK-BB, and an anti-CK-B antibody which recognizes a region of the 1st to 100th amino acid from N-terminus in the amino acid sequence of the subunit B of creatine kinase.

10. The kit according to claim 9, wherein the first antibody and the second antibody recognize both the subunit M and the subunit B of CK-MB.

11. The kit according to claim 9, comprising a first reagent containing the anti-CK-B antibody, and a second reagent containing the first antibody and the second antibody.

12. The kit according to claim 9, wherein the region recognized by the anti-CK-B antibody is the one having an amino acid sequence shown in SEQ ID NO: 1.

13. The kit according to claim 9, wherein the first antibody and/or the second antibody are immobilized on an insoluble carrier.

14. The kit according to claim 13, wherein the insoluble carrier is a latex particle.

## Patentansprüche

1. Verfahren zur Messung von Kreatinkinase-MB-Isozym (im folgenden als CK-MB abgekürzt) in einer Probe, umfassend:
(1) einen Schritt zum Reagieren der Probe mit einem Anti-CK-B-Antikörper, der eine Region der 1. bis 100. Aminosäure vom N-Terminus aus in der Aminosäuresequenz einer Untereinheit B der Kreatinkinase erkennt, und dann Reagieren mit einem ersten Antikörper gegen CK-MB und einem zweiten Antikörper gegen CK-MB, der ein anderes Epitop erkennt als der erste Antikörper gegen CK-MB,
wobei der erste Antikörper und der zweite Antikörper nicht nur mit CK-MB, sondern auch mit einem Kreatinkinase-BB-Isoenzym (nachfolgend als CK-BB abgekürzt) reagiert (Schritt 1);
(2) einen Schritt zum Messen einer durch die Reaktion erzeugten optischen Veränderung (Schritt 2); und
(3) einen Schritt zur Bestimmung einer Menge an CK-MB in der Probe auf der Grundlage des in Schritt 2 (Schritt 3) erhaltenen Ergebnisses.

2. Verfahren nach Anspruch 1, wobei der erste Antikörper und der zweite Antikörper sowohl eine Untereinheit M als auch die Untereinheit B von CK-MB erkennen.

3. Verfahren nach Anspruch 1, wobei die durch den Anti-CK-B-Antikörper zu erkennende Region diejenige mit einer in SEQ ID NO: 1 gezeigten Aminosäuresequenz ist.

4. Verfahren nach Anspruch 1, wobei die optische Änderung Absorptionsänderung, Veränderung der Trübung oder Veränderung der Streulichtintensität ist.

5. Verfahren nach Anspruch 1, wobei der erste Antikörper und/oder der zweite Antikörper auf einem unlöslichen Träger immobilisiert werden.

6. Verfahren nach Anspruch 5, wobei der unlösliche Träger ein Latexteilchen ist.

7. Verfahren zur Vermeidung eines Einflusses von CK-BB bei der Messung von CK-MB mit einem ersten Antikörper gegen CK-MB und einem zweiten Antikörper gegen CK-MB, der ein anderes Epitop als das des ersten Antikörpers erkennt,
worin der erste Antikörper und der zweite Antikörper nicht nur mit CK-MB, sondern auch mit CK-BB reagieren, umfassend die Behandlung einer Probe mit einem Anti-CK-B-Antikörper, der eine Region der 1. bis 100. Aminosäure vom N-Terminus aus in der Aminosäuresequenz der Untereinheit B der Kreatinkinase erkennt.

8. Verfahren nach Anspruch 7, wobei die CK-MB-Messung durchgeführt wird unter Verwendung eines ersten Antikörpers gegen CK-MB und eines zweiten Antikörpers gegen CK-MB, der ein anderes Epitop als das des ersten Antikörpers erkennt, und die Messung durch CK-BB beeinflusst wird.

9. Kit zur Messung von CK-MB, umfassend einen ersten Antikörper gegen CK-MB, einen zweiten Antikörper gegen CK-MB, der ein anderes Epitop als das des ersten Antikörpers erkennt,
worin der erste Antikörper und der zweite Antikörper nicht nur mit CK-MB, sondern auch mit CK-BB reagieren, und einen Anti-CK-B-Antikörper, der eine Region der 1. bis 100. Aminosäure vom N-Terminus aus in der Aminosäuresequenz der Untereinheit B der Kreatinkinase erkennt.

10. Kit nach Anspruch 9, wobei der erste Antikörper und der zweite Antikörper sowohl die Untereinheit M als auch die Untereinheit B von CK-MB erkennen.

11. Kit nach Anspruch 9, umfassend ein erstes Reagenz, das den Anti-CK-B-Antikörper enthält, und ein zweites Reagenz, das den ersten Antikörper und den zweiten Antikörper enthält.

12. Kit nach Anspruch 9, wobei die durch den Anti-CK-B-Antikörper erkannte Region diejenige mit einer in SEQ ID NO: 1 gezeigten Aminosäuresequenz ist.

13. Kit nach Anspruch 9, wobei der erste Antikörper und/oder der zweite Antikörper auf einem unlöslichen Träger immobilisiert sind.

14. Kit nach Anspruch 13, wobei der unlösliche Träger ein Latexteilchen ist.

## Revendications

1. Procédé de mesure d'isoenzyme de créatine kinase MB (ci-après abrégée par CK-MB) dans un échantillon, comprenant :
(1) une étape de réaction de l'échantillon avec un anticorps anti-CK-B qui reconnaît une zone allant du 1^{er} au 100^{ième} acide aminé à partir de la terminaison N dans la séquence d'acides aminés d'une sous-unité B de créatine kinase, et ensuite une réaction avec un premier anticorps dirigé contre CK-MB et un second anticorps dirigé contre CK-MB qui reconnaît un épitope différent de celui reconnu par le premier anticorps dirigé contre CK-MB,
dans lequel le premier anticorps et le second anticorps réagissent non seulement avec CK-MB mais également avec une isoenzyme de créatine kinase BB (ci-après abrégée par CK-BB) (étape 1) ;
(2) une étape de mesure d'un changement optique généré par la réaction (étape 2) ; et
(3) une étape de détermination d'une quantité de CK-MB dans l'échantillon sur la base du résultat obtenu dans l'étape 2 (étape 3).

2. Procédé selon la revendication 1, dans lequel le premier anticorps et le second anticorps reconnaissent tous les deux une sous-unité M et la sous-unité B de CK-MB.

3. Procédé selon la revendication 1, dans lequel la zone à reconnaître par l'anticorps anti-CK-B est celle ayant une séquence d'acides aminés représentée par SEQ ID NO : 1.

4. Procédé selon la revendication 1, dans lequel le changement optique est un changement d'absorbance, un changement de turbidité, ou un changement d'intensité de lumière diffusée.

5. Procédé selon la revendication 1, dans lequel le premier anticorps et/ou le second anticorps sont immobilisés sur un support insoluble.

6. Procédé selon la revendication 5, dans lequel le support insoluble est une particule de latex.

7. Procédé pour éviter une influence de CK-BB dans la mesure de CK-MB avec un premier anticorps dirigé contre CK-MB et un second anticorps dirigé contre CK-MB qui reconnaît un épitope différent de celui reconnu par le premier anticorps,
dans lequel le premier anticorps et le second anticorps réagissent non seulement avec CK-MB mais également avec CK-BB, comprenant le traitement d'un échantillon avec un anticorps anti-CK-B qui reconnaît une zone allant du 1^{er} au 100^{ième} acide aminé à partir de la terminaison N dans la séquence d'acides aminés de la sous-unité B de créatine kinase.

8. Procédé selon la revendication 7, dans lequel la mesure de CK-MB est réalisée en utilisant un premier anticorps dirigé contre CK-MB et un second anticorps dirigé contre CK-MB qui reconnaît un épitope différent de celui reconnu par le premier anticorps, et la mesure est influencée par CK-BB.

9. Kit pour la mesure de CK-MB comprenant un premier anticorps dirigé contre CK-MB, un second anticorps dirigé contre CK-MB qui reconnaît un épitope différent de celui reconnu par le premier anticorps, dans lequel le premier anticorps et le second anticorps réagissent non seulement avec CK-MB mais également avec CK-BB, et un anticorps anti-CK-B qui reconnaît une zone allant du 1^{er} au 100^{ième} acide aminé à partir de la terminaison N dans la séquence d'acides aminés de la sous-unité B de créatine kinase.

10. Kit selon la revendication 9, dans lequel le premier anticorps et le second anticorps reconnaissent tous les deux la sous-unité M et la sous-unité B de CK-MB.

11. Kit selon la revendication 9, comprenant un premier réactif contenant l'anticorps anti-CK-B, et un second réactif contenant le premier anticorps et le second anticorps.

12. Kit selon la revendication 9, dans lequel la zone reconnue par l'anticorps anti-CK-B est celle ayant une séquence d'acides aminés représentée par SEQ ID NO : 1.

13. Kit selon la revendication 9, dans lequel le premier anticorps et/ou le second anticorps sont immobilisés sur un support insoluble.

14. Kit selon la revendication 13, dans lequel le support insoluble est une particule de latex.
